Europäisches Patentamt

European Patent Office

Office européen des brevets

(19)

(11) Publication number: **0 052 861**
**B1**

(12) **EUROPEAN PATENT SPECIFICATION**

(45) Date of publication of patent specification: **16.05.84**

(51) Int. Cl.³: **A 61 K 45/02,** C 12 P 21/00 // C12N15/00

(21) Application number: **81109770.8**

(22) Date of filing: **19.11.81**

(54) Extraction of interferon from bacteria.

(30) Priority: **26.11.80 US 210426**
**29.12.80 US 221135**

(43) Date of publication of application:
**02.06.82 Bulletin 82/22**

(45) Publication of the grant of the patent:
**16.05.84 Bulletin 84/20**

(84) Designated Contracting States:
**AT BE CH DE FR GB IT LI NL SE**

(56) References cited:
**US-A-4 262 090**

**NATURE, vol. 287, no. 5781, October 1980, Chesham, Bucks, (GB) D.V. GOEDDEL et al.: "Human leukocyte interferon produced by E. coli is biologically active", pages 411-416 NATURE, vol. 292, August 20, 1981 H. SCHELLEKENS et al.: "Comparative antiviral efficiency of leukocyte and bacterially produced human alpha-interferon in rhesus monkeys", pages 775-776**

(73) Proprietor: **SCHERING CORPORATION**
**2000 Galloping Hill Road**
**Kenilworth, New Jersey 07033 (US)**

(72) Inventor: **Leibowitz, Paul**
**185 Prospect Avenue**
**Hackensack New Jersey 07601 (US)**
Inventor: **Weinstein, Marvin J.**
**26 Tanglewood Lane**
**East Brunswick New Jersey 08816 (US)**

(74) Representative: **Antony, Fritz, Dr. et al**
**P.O. Box 601 Winkelriedstrasse 35**
**CH-6002 Lucerne (CH)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

The present invention relates to extraction of interferon from interferon-expressing bacteria.

Although interferon is widely believed to be of great importance as a potential anti-viral and/or anti-cancer agent, the scarcity and great expense of interferon obtained from natural sources has delayed substantial clinical testing and widespread use of interferon. Recombinant DNA techniques have been used to create bacteria capable of expressing interferon; see, for example, Nagata et al., "Nature", vol. 284, 316—320 (1980). Fermentation of such bacteria is expected to yield large quantities of interferon at substantially lower cost than would be possible by using natural sources of interferon. However, interferon for clinical use must be of high purity and not be contaminated by cell constituents or cell debris of the interferon-expressing bacteria. Contamination by such impurities could result in adverse reactions or in test results that are not reproducible. Accordingly, extraction of interferon from the cells of interferon-expressing bacteria in sufficiently high purity for clinical use has presented a major problem.

We have now surprisingly discovered an improved method for extracting interferon from interferon-expressing bacteria.

According to the invention we provide a method of obtaining a solution of interferon from interferon-expression bacterial cells comprising acidifying a first suspension of interferon-containing bacterial cells, removing substantially all of the suspending liquid from the cells, preparing a second suspension of the acidified cells, at least neutralissing said second suspension and separating the interferon-containing liquid from the suspended cells. If desired, the resulting liquid can be further processed, in particular by extracting the interferon from said liquid, e.g. by known methods.

The phrase "at least neutralising" in the foregoing paragraph indicates that the second suspension is rendered neutral or weakly alkaline. For convenience, the word "neutralising" alone will be used henceforth in this specification and in the claims to indicate this.

By the method of the present invention, interferon is surprisingly released from the cells on neutralising the suspension of acidified cells, without the need for mechanical or enzymatic disruption of the cell surface. This method allows efficient recovery of interferon in a manner which significantly reduces contamination by cell constituents and makes subsequent purification steps easier and less expensive.

In the method of the present invention, the suspension of interferon-expressing cells, which may be in a fermentation tank, is preferably acidified to a pH of about 1.3 to 4.0, more preferably to a pH of about 2.0 to 2.5. Examples of suitable acids that may be used in the acidification step are strong acids, especially mineral acids such as hydrochloric acid, nitric acid, sulfuric acid and phosphoric acid. However, it is generally preferred not to use acids (such as hydrochloric acid) that may corrode the fermentation equipment used to culture the cells. Sulfuric acid and phosphoric acid are therefore most preferred. Preferably, the aforementioned suspending media are aqueous.

The temperature range at which the bacterial cells are treated with acid should be from about ambient temperature (that it, not lower than about 15°C.) to about 40°C. If the temperature is too low, the bacteria will not be killed fast enough during the acid treatment. The maximum temperature is about 40°C. because the interferon may be degraded at higher temperatures.

After the cell suspension has been acidified, the cells are removed from the liquid, preferably by centrifugation. If the cells are centrifuged to form a pellet they should be resuspended before the step of neutralising. If the centrifuge is designed to use a portion of the suspending liquid to form a slurry of the centrifuged cells, there may be sufficient liquid in the slurry for one to proceed to the step of neutralising without resuspending the cells. The word "suspension" as used herein includes such a slurry.

Preferably, the cell suspension is neutralised to a pH of about 7.0 to 8.0, preferably 7.2 to 7.6. Examples of suitable bases that may be used in the step of neutralising are potassium hydroxide and sodium hydroxide.

Examples of bacteria that can be altered by recombinant DNA techniques to produce interferon and from which interferon may then be extracted using the method of the present invention are E. coli, Bacillus subtilis, actinomycetes and the like. The preferred bacteria are E. coli and Bacillus subtilis. E. coli are most preferred.

The method of the present invention can in particular be used with bacteria that express leucocyte interferon; such bacteria can be prepared for example by the method of Nagata et al., "Nature", vol. 284, 316—320 (1980). The method of the present invention may also be used with bacteria that express fibroblast interferon; such bacteria can be prepared for example by the method of Derynck et al., "Nature", vol. 287, 193—197 (1980). The method of the present invention is especially useful with bacteria that express human leucocyte interferon.

Leucocyte and fibroblast interferons have also been referred to by the nomenclature IFN-alpha and IFN-beta respectively. Each of the aforementioned types of interferons may also have different forms. See, for example, UK Patent Application 2,037,296A, published July 9, 1980; Streuli et al., "Science", vol. 209, pages 1343—1347 (1980); Goedel et al., "Nature", vol. 287, pages 411—416 (1981);

and Streuli *et al., Proc. Natl. Acad. Sci. U.S.A.,* vol 78, pages 2848—2852 (1981) (Biochemistry).

The following Example illustrates but does not limit the invention:

*Example*

Add 100 ml. of an innoculum of interferon-expressing HB—101 *E. coli* containing the plasmid Z—pBR 322 (Pst)/HcIF—SN—35 or a derivative thereof, prepared by the method of Nagata *et al.,* "Nature", vol. 284, 316—320 (1980), to 9.9 liters of broth in a 14 liter fermentor at 37°C. The broth is prepared from 330 g. Cerelose (Trade Mark), 310 g. yeast extract, 50 g. $KH_2PO_4$, 10 g. $MgSO_4.7H_2O$ and water to make 9.9 liters.

Maintain the pH at 7.0 and the temperature at 37°C. while aerating and agitating the mixture until the culture's growth reaches late exponential (5 to 7 hours). Then add 10 N phosphoric acid to the *E. coli* suspension in the fermentor until a pH of 2.1 is obtained and allow the mixture to stand for one hour while maintaining the temperature at 25 to 37°C. Periodically, recheck the pH of the mixture and, if necessary, readjust it to 2.1. Then harvest the contents of the fermentor and centrifuge them at about 5000xg or above.

After centrifugation, harvest the bacterial pellet. Resuspend the pellet as an approximately 10% weight/volume suspension of bacteria in an aqueous solution that is 50 mM Tris(2 - amino - 2 - hydroxymethyl - 1,3 - propanediol) and 0.15 M sodium chloride (pH7.5) and add 3 N sodium hydroxide until a pH of about 7.3 is obtained. (If the centrifuge that is available produces a slurry rather than a pellet, it may not be necessary to resuspend the product in the aforementioned aqueous solution and one may proceed to add 3 N sodium hydroxide to the slurry). Mix the resulting suspension for one hour at 4°C. and then centrifuge at 5000xg or above. Discard the pellet. The supernatant contains extracted interferon, specifically α1 interferon (INF-α1), as defined by Streuli *et al.,* "Science", vol. 209 pages 1343—1347 (1980), which may be further purified or concentrated. The presence of interferon in the supernatant is indicated by methods disclosed in "The Interferon System" by William E. Stewart II, Springer Verlag, New York, pages 134—156 (1979).

Similarly, after using a plasmid expressing α2 interferon, as defined by Streuli *et al.,* "Science", vol. 209, 1343—1347 (1980), extract such interferon from the culture broth.

Similarly, after using a plasmid expressing another type of leucocyte interferon (for example, an interferon selected from the interferons defined in UK Patent Application 2,037,296A, published July 9, 1980) extract that interferon from the culture broth.

Similarly, after using a plasmid expressing fibroblast interferon, extract that interferon from the culture broth.

Derivatives of the aforementioned plasmid Z—pBR 322 (Pst)/HcIF—SN—35 that express IFN—α1 more efficiently may be prepared. The method of the present invention is likewise applicable to extraction of interferon from bacteria containing such plasmids.

**Claims**

1. A method of obtaining a solution of interferon from interferon-expressing bacterial cells comprising acidifying a first suspension of interferon-containing bacterial cells, removing substantially all of the suspending liquid from the cells, preparing a second suspension of the acidified cells, at least neutralising said second suspension and separating the interferon-containing liquid from the suspended cells.

2. A method of obtaining interferon from interferon-expressing bacterial cells comprising acidifying a first suspension of interferon-containing bacterial cells, removing substantially all of the suspending liquid from the cells, preparing a second suspension of the acidified cells, at least neutralising said second suspension, separating the interferon-containing liquid from the suspended cells and extracting the interferon from said liquid.

3. A method as claimed in claims 1 or 2 wherein said first suspension is acidified with a strong mineral acid, preferably with sulfuric acid or phosphoric acid.

4. A method as claimed in any of claims 1 to 3 wherein said first suspension is acidified to a pH of about 1.3 to 4.0, preferably to about 2.0 to 2.5.

5. A method as claimed in any of claims 1 to 4 wherein said second suspension is neutralized with potassium hydroxide or sodium hydroxide.

6. A method as claimed in any of claims 1 to 5, wherein said second suspension is neutralized to a pH of about 7.0 to 8.0, preferably about 7.2 to 7.6.

7. A method as claimed in any of claims 1 to 6 wherein said bacterial cells are *E. coli* cells.

8. A method as claimed in any of claims 1 to 7 wherein the interferon is leucocyte interferon.

**Revendications**

1. Méthode pour obtenir une solution d'interféron à partir de cellules bactériennes exprimant 1'interféron consistant à acidifier une première suspension de cellules bactériennes contenant de l'interféron, à enlever sensiblement tout le liquide de suspension des cellules, à préparer une seconde suspension de cellules acidifiées, à neutraliser au moins ladite seconde suspension et à séparer la liquide contenant l'interféron des cellules en suspension.

2. Méthode pour obtenir un interféron à partir de cellules bactériennes exprimant l'interféron consistant à acidifier une premier suspension de cellules bactériennes contenant de l'interféron, à enlever sensiblement tout le liquide de suspension des cellules, à préparer une seconde suspension de cellules acidifiées, à neutraliser au moins ladite seconde suspension, à séparer le liquide contenant l'interféron des cellules en suspension et à extraire l'interféron dudit liquide.

3. Méthode selon la revendication 1 ou 2, où ladite première suspension est acidifiée avec un acide minéral fort, de préférence avec de l'acide sulfurique ou de l'acide phosphorique.

4. Méthode selon l'une des revendications 1 à 3, où ladite première suspension est acidifiée à un pH d'environ 1,3 à 4,0, de préférence d'environ 2,0 à 2,5.

5. Méthode selon l'une des revendications 1 à 4, où ladite seconde suspension est neutralisée avec de la potasse ou de la soude.

6. Méthode selon l'une des revendications 1 à 5, où ladite seconde suspension est neutralisée à un pH d'environ 7,0 à 8,0, de préférence d'environ 7,2 à 7,6.

7. Méthode selon l'une des revendications 1 à 6, où les cellules bactériennes sont des cellules de *E. coli*.

8. Méthode selon l'une des revendications 1 à 7, où l'interféron est l'interféron de leucocytes.

**Patentansprüche**

1. Verfahren zur Gewinnung einer Lösung von Interferon aus Interferon bildenden bakterillen Zellen, umfassend das Ansäuern einer ersten Suspension Interferon enthaltender bakterieller Zellen, das Entfernen im wesentlichen der Gesamtmenge der suspendierenden Flüssigkeit von den Zellen, das Herstellen einer zweiten Suspension der angesäuerten Zellen, wenigstens das Neutralisieren der zweiten Suspension sowie die Abtrennung der Interferon-haltigen Flüssigkeit von den suspendierten Zellen.

2. Verfahren zur Gewinnung von Interferon aus Interferon bildenden bakteriellen Zellen, umfassend das Ansäuern einer ersten Suspension Interferon enthaltender bakterieller Zellen, das Entfernen im wesentlichen der Gesamtmenge der suspendierenden Flüssigkeit von den Zellen, das Herstellen einer zweiten Suspension der angesäuerten Zellen, wenigstens das Neutralisieren der zweiten Suspension, die Abtrennung der Interferon-haltigen Flüssigkeit von den suspendierten Zellen sowie die Extraktion des Interferons aus der Flüssigkeit.

3. Verfahren nach Anspruch 1 oder 2, dadurch gekennzeichnet, daß die erste Suspension mit einer starken Mineralsäure, vorzugsweise mit Schwefelsäure oder Phosphorsäure, angesäuert wird.

4. Verfahren nach Anspruch 1 bis 3, dadurch gekennzeichnet, daß die erste Suspension auf einen pH von etwa 1,3 bis 4,0, vorzugsweise von etwa 2,0 bis 2,5, angesäuert wird.

5. Verfahren nach Anspruch 1 bis 4, dadurch gekennzeichnet, daß die zweite Suspension mit Kaliumhydroxid oder Natriumhydroxid neutralisiert wird.

6. Verfahren nach Anspruch 1 bis 5, dadurch gekennzeichnet, daß die zweite Suspension auf einen pH von etwa 7,0 bis 8,0, vorzugsweise von etwa 7,2 bis, 7,6, neutralisiert wird.

7. Verfahren nach Anspruch 1 bis 6, dadurch gekennzeichnet, daß die bakteriellen Zellen Zellen von E. coli sind.

8. Verfahren nach Anspruch 1 bis 7, dadurch gekennzeichnet, daß das Interferon Leukozyten-Interferon ist.